(19) Europäisches Patentamt
European Patent Office
Office européen des brevets

(11) Numéro de publication: **0 120 757**
**A1**

(12) # DEMANDE DE BREVET EUROPEEN

(21) Numéro de dépôt: **84400481.2**

(51) Int. Cl.³: **A 61 K 9/50**

(22) Date de dépôt: **09.03.84**

(30) Priorité: **11.03.83 FR 8304037**

(43) Date de publication de la demande: **03.10.84 Bulletin 84/40**

(84) Etats contractants désignés: **AT BE CH DE FR GB IT LI LU NL SE**

(71) Demandeur: **Etablissement Public dit: CENTRE NATIONAL DE LA RECHERCHE SCIENTIFIQUE (CNRS)**
**15, Quai Anatole France**
**F-75007 Paris(FR)**

(71) Demandeur: **UNIVERSITE DE HAUTE NORMANDIE**

**F-76130 Mont Saint Aignan(FR)**

(72) Inventeur: **Vert, Michel René**
**19, rue de Bargteheide**
**F-76250 Deville-les-Rouen(FR)**

(72) Inventeur: **Huguet, née Luzaic Jovanka**
**8, Allée Primevère**
**F-76840 Henouville(FR)**

(72) Inventeur: **Braud, Christian Gérard**
**10, rue des Aubépines**
**F-76420 Bihorel(FR)**

(72) Inventeur: **Bouffard, Philippe**
**21, rue de la Convention**
**F-76620 Le Havre(FR)**

(72) Inventeur: **Clabaut née Boquet, Monique Thérèse**
**2, rue du Docteur Mathieu**
**F-76710 Montville(FR)**

(72) Inventeur: **Delpech, Bertrand**
**89, avenue Pasteur**
**F-76410 Saint Aubin les Elbeuf(FR)**

(74) Mandataire: **Nony, Michel**
**Cabinet Nony 29, rue Cambacérès**
**F-75008 Paris(FR)**

(54) **Nouvelles compositions sous forme de microglobules à base de polyamines tertiaires partiellement quaternisées, contenant des substances hydrophobes, leur préparation et leur application.**

(57) Composition sous forme de microglobules solubles dans l'eau.

Lesdits globules sont constitués par au moins une polyamine tertiaire partiellement quaternisée, dans lesdits microglobules est incorporée au moins une substance hydrophobe, ladite polyamine tertiaire partiellement quaternisée dérive d'une polyamine tertiaire insoluble dans l'eau, soluble en milieu acide, et présentant un fort pouvoir tampon en milieu aqueux; et procédé de solubilisation de substances hydrophobes.

EP 0 120 757 A1

0120757

NOUVELLES COMPOSITIONS SOUS FORME DE MICROGLOBULES A BASE DE POLYAMINES TERTIAIRES PARTIELLEMENT QUATERNISEES, CONTENANT DES SUBSTANCES HYDROPHOBES, LEUR PREPARATION ET LEUR APPLICATION.

La présente invention a pour objet de nouvelles compositions, sous forme de microglobules à base de polyamines tertiaires partiellement quaternisées et de substances hydrophobes, leur préparation et leur application.

On sait que l'utilisation de certaines substances intéressantes notamment dans le domaine des médicaments, des colorants, des cosmétiques, etc... est limitée par la faible solubilité dans les systèmes aqueux de ces substances lorsqu'elles présentent un caractère hydrophobe.

Par exemple, les techniques de solubilisation des drogues constituent une branche d'importance croissante de la pharmacie galénique (dans la présente demande, on appelle "drogue" la substance active d'un médicament).

On sait aussi que de nombreuses recherches portent actuellement sur le transport des drogues jusqu'à leur site d'action dans l'organisme pour favoriser l'activité des médicaments tout en diminuant les effets secondaires.

De nombreux systèmes vecteurs ont été proposés, parmi lesquels on peut citer des anticorps, des protéines, des enzymes, des hormones, des liposomes, des polymères, etc...; voir par exemple G. Grégoriadis, The Lancet, August 1, 1981, page 241.

Dans le domaine des macromolécules synthétiques on a jusqu'à présent surtout cherché des systèmes fondés sur un accrochage temporaire des substances actives par liaisons covalentes labiles. Dans le cas d'un médicament, ces systèmes présentent l'inconvénient de constituer autant de drogues nouvelles dont l'homologation doit être obtenue, avec tous les frais importants que cela implique.

Pour cette raison, les systèmes transporteurs agissant par microencapsulation physique constituent une solution particulièrement intéressante. On peut effectuer soit une microencapsulation par enrobage avec un solide soit une microencapsulation de type micellaire. Dans ce cas, les substances actives occluses sont libérées par diffusion ou par dégradation des enveloppes. Dans ce domaine, les liposomes ont fait l'objet de nombreuses recherches pour tenter de remédier à leurs inconvénients connus, c'est-à-dire leur instabilité et les "fuites" de substances actives qui sont observées.

La présente invention repose sur l'utilisation de systèmes macromoléculaires synthétiques constitués par des polybases (polyamines tertiaires partiellement quaternisées) comportant des parties hydrophiles et des parties hydrophobes présentes en proportions variables selon l'état d'ionisation (fonction des réactions acide-base) et disposées de manière aléatoire le long des chaînes macromoléculaires de telle sorte que lesdites macromolécules prennent, dans l'eau, des conformations particulières compactes

pouvant constituer des microphases (ou microglobules) lipophiles finement dispersées et stables. On a découvert que ces microphases sont capables d'emprisonner et de retenir des substances hydrophobes (réalisant ainsi une microencapsulation moléculaire) et de les disperser de manière telle qu'on obtient un effet de solubilisation dans les milieux aqueux. On a également découvert que ces microphases peuvent être déstabilisées par modification de l'équilibre acide-base, et que lesdites substances hydrophobes peuvent être alors libérées soudainement.

L'invention a pour objet une composition sous forme de microglobules solubles dans l'eau, caractérisée par le fait que lesdits globules sont constitués par au moins une polyamine tertiaire partiellement quaternisée, que dans lesdits microglobules est incorporée au moins une substance hydrophobe, et que ladite polyamine tertiaire partiellement quaternisée dérive d'une polyamine tertiaire (non quaternisée) insoluble dans l'eau, soluble en milieu acide, et présentant un fort pouvoir tampon en milieu aqueux.

On sait que le fort pouvoir tampon se traduit sur les courbes de titrage potentiométrique par un plateau de pH important. Les polyamines tertiaires dont dérivent les polyamines partiellement quaternisées utilisées selon l'invention sont donc des polyamines tertiaires dont les courbes de titrage potentiométrique en milieu aqueux présentent un plateau de pH dans une large gamme de degrés de protonation apparents, généralement sur une gamme d'au moins 30% (et de préférence d'au moins 50%) de degré de protonation apparent.

Les propriétés des microglobules utilisés dans les compositions de l'invention ne dépendent pas du poids moléculaire des polyamines tertiaires, à condition que celles-ci comportent au moins une vingtaine de motifs. De préférence, le poids moléculaire, qui peut être mesuré selon les cas par osmométrie, par diffusion de la lumière ou par mesure de la viscosité, est supérieur à 5000. Il peut varier notamment de 5000 à plusieurs millions (par exemple 10 millions).

Bien entendu, la substance hydrophobe présente dans la composition de l'invention est une substance qui n'interagit pas avec les groupes ioniques de la polyamine tertiaire partiellement quaternisée, ou qui n'interagit pas substantiellement avec lesdits groupes ioniques, car il faut que la fixation de cette substance soit due aux interactions hydrophobes, et non aux interactions ioniques.

La composition de l'invention peut se présenter soit sous la forme d'une solution aqueuse, soit sous la forme d'un produit lyophilisé qui peut être facilement solubilisé en milieu aqueux.

La notion de substance hydrophobe est connue; on considère généralement qu'une substance hydrophobe est une substance réputée insoluble en milieu aqueux et soluble dans des milieux organiques.

Les substances hydrophobes peuvent être en particulier :

- des drogues telles que par exemple des hormones stéroïdes synthétiques ou naturelles (progestérone, estradiol, etc...) des antibiotiques (par exemple griseofulvine), des drogues anti-cancer telles que nitrosourées (CCNU), fluorouracile, azathioprine etc... ;

- des colorants tels que par exemple l'o-tolylazo-bêta-naphtylamine, etc... ;

- des substances aromatiques lipophiles capables d'absorber l'ultra-violet telles que notamment des esters de l'acide p-amino ou p-diméthylamino benzoïque (par exemple p-diméthylaminobenzoate d'amyle), des esters salicyliques (salicylate d'homomenthyle), le cinnamate de benzyle, etc...

- des substances odorantes utilisables comme parfums telles que par exemple le diphénylméthane, la jasmone, la dihydro-isojasmone, etc...

Les proportions relatives de substance hydrophobe et de polybase partiellement quaternisée, dans les compositions de l'invention, varient avec lesdites substances et lesdites polybases. La quantité maximum de substance hydrophobe qu'il est possible de solubiliser grâce auxdites compositions peut être facilement déterminée expérimentalement dans chaque cas, comme cela est montré dans les exemples ci-après.

Généralement, la proportion pondérale de substance hydrophobe, par rapport à la polybase partiellement quaternisée, varie de 0,01 à 100%, en particulier de 0,01 à 50% et le plus souvent de 0,1 à 20%.

Dans ce qui suit, on fera plus particulièrement référence à l'utilisation des polyamines tertiaires partiellement quaternisées pour la solubilisation et le transport de drogues hydrophobes mais il est bien entendu que ces polyamines partiellement quaternisées peuvent être utilisées pour solubiliser toutes substances hydrophobes telles que définies ci-dessus.

Les groupements amine quaternisés, qui constituent les parties hydrophiles de la molécule de polybase, doivent être (statistiquement) suffisamment éloignés les uns des autres pour que la chaîne située entre deux atomes d'azote quaternaire consécutifs présente un caractère hydrophobe.

D'autre part, le polymère doit comporter suffisamment de parties hydrophiles stables (motifs avec atome d'azote quaternisé) pour être hydrosoluble, ou hydrodispersable sous forme de solution colloïdale stable.

Les polyamines tertiaires partiellement quaternisées formant des microphases globulaires utilisables selon l'invention ont un degré de quaternisation d'au moins 2%, pouvant varier en particulier de 3% à 30%.

Parmi les polyamines tertiaires dont dérivent les polybases partiellement quaternisées utilisées selon l'invention, on citera par exemple: les polyamines partiellement quaternisées dérivant de polyamines tertiaires insolubles dans l'eau (mais solubles en milieu acide) comportant des groupements amine tertiaire aliphatiques ou aromatiques, et notamment des polymères portant des substituants de formule:

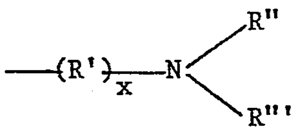

dans laquelle:

x représente le nombre zéro ou 1,

R' est un groupement divalent de type aliphatique, aryle ou arylaliphatique, pouvant être substitué et/ou comporter des hétéroatomes, ayant jusqu'à 18 atomes de carbone,

R" et R"' représentent un groupement aliphatique, aryle ou arylaliphatique, pouvant être substitué ou comporter des hétéroatomes, ayant jusqu'à 18 atomes de carbone,

étant entendu que (R' et R") ou (R" et R"') peuvent représenter ensemble un cycle aliphatique ou aromatique incluant comme hétéroatome l'atome d'azote auquel ils sont attachés, ledit cycle pouvant comporter d'autres hétéroatomes.

En particulier, ces substituants peuvent être du type:

$-(R)_x-NR_1(R_2)$

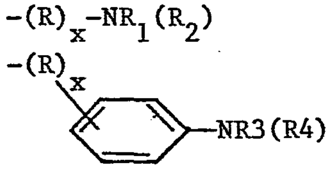

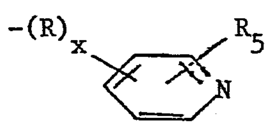

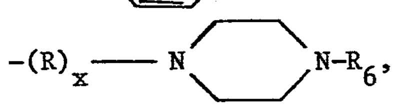, etc...,

x étant défini comme ci-dessus, R étant un groupement aliphatique divalent, tel qu'un groupement alkylène, éventuellement substitué, ayant par exemple 1 à 18 atomes de carbone, $R_1$, $R_2$, $R_3$, $R_4$, $R_5$ et $R_6$ étant par exemple des groupements aliphatiques, notamment alkyle, éventuellement substitués, ayant 1 à 18 atomes de carbone ou des groupements aralkyle, en particulier phénylalkyle, éventuellement substitués dont le groupement alkyle a 1 à 8 atomes de carbone, tels que des groupements benzyle ou phénéthyle, ou $R_1$ et

$R_2$, ou bien $R_3$ et $R_4$ représentant ensemble, avec l'atome d'azote auquel ils sont liés, un cycle à 5 ou 6 chaînons pouvant comporter d'autres hétéroatomes. Les divers substituants éventuellement présents sur les groupements R et $R_1$-$R_6$ peuvent être quelconques à condition qu'ils ne réagissent pas chimiquement avec la substance hydrophobe et qu'ils ne présentent pas un caractère ionique.

Parmi lesdites polyamines tertiaires, on citera en particulier:

a) les poly [(N,N-dialkylaminoalkyl) éthylène] et les poly [thio (N,N-dialkylaminoalkyl)-1 éthylène] dont les groupements alkyle ont 1 à 18 atomes de carbone, et notamment le poly [(N-sec-butyl N-méthylaminométhyl) éthylène] appelé en abrégé P(BMAE), le poly [thio(N-méthyl N-phényl-1-éthylaminométhyl)-1 éthylène], le poly [thio (N-s-butyl-N-méthylamino-méthyl)-1 éthylène], appelé en abrégé P(TBMAE); le poly [thio(N,N-diéthyl-aminométhyl-1 éthylène], appelé en abrégé P(TDAE); de tels polymères sont connus ou peuvent être préparés selon les méthodes connues. Par exemple, les poly [thio(N,N-dialkylaminométhyl)-1 éthylène] peuvent être préparés selon les méthodes mentionnées dans les articles suivants: J. Huguet M. Vert, N. Spassky, and E. Selegny, Makromol. Chem.170,23 (1973) J. Huguet, M. Vert and E. Selegny Eur. Polym.J, 10, 261 (1974), J. Huguet and M. Vert, Eur. Polym. J. 12, 469 (1976) et J. Huguet, Nouv. J. Chimie, 3, 293 (1979); les poly [(N,N-dialkylaminoalkyl) éthylène] peuvent être préparés notamment par réduction des poly(N,N-dialkyl acrylamides) ou des polyamides homologues par exemple avec un hydrure $LiAlH_4$; voir par exemple F. DANUSSO and P. FERRUTI, Polymer, 11, 88 (1970).

b) les poly [(N,N-dialkylaminoalkyl acrylates (ou méthacrylates)]; voir par exemple A. Shatkay and I. Michaeli, J. Phys. Chem. 70, 3777 (1966).

Ces polyamines tertiaires insolubles dans l'eau sont partiellement quaternisées par exemple par l'action en quantité mesurée d'un agent de quaternisation classique, notamment un agent d'alkylation tel que le sulfate de diméthyle, l'iodure de méthyle, etc...

On désigne les polyamines partiellement quaternisées par le préfixe Q et on indique le degré de quaternisation comme indiqué par l'exemple suivant: Q-PTDAE17 représente un polymère PTDAE partiellement quaternisé à un taux de 17%; voir par exemple D. VALLIN, J. HUGUET and M. VERT, Polymer Journal, Vol. 12 n°2, 113-124 (1980) et J. HUGUET, D. VALLIN and M. VERT, Polymer Journal, vol. 14, n°5, 335-342 (1982).

c) les polymères composés de motifs de formule :

$$-HN-(CH_2)_4-\underset{\displaystyle N(Alk.)_2}{\underset{|}{CH}}-CO-$$

dans laquelle Alk. est un groupement alkyle ayant par exemple 1 à 4 atomes de carbone. La préparation de ces polymères, et leur quaternisation à l'aide d'un

agent d'alkynation tel que ceux mentionnés précédemment, peuvent être effectués par exemple selon la méthode décrite dans la demande de brevet japonais Kokai n° 58-86085.

Les polyamines tertiaires partiellement quaternisées telles que définies précédemment peuvent prendre des conformations globulaires, soit monomoléculaires, soit agrégées (quand le degré de quaternisation est faible), à pH alcalin ou voisin de la neutralité. Ces globules correspondent à une conformation compacte de la macromolécule, les parties hydrophobes constituant une microphase organique stabilisée par les charges permanentes des atomes d'azote quaternisées qui se trouvent à la surface du globule. Cette microphase organique est capable de dissoudre les substances hydrophobes et d'en augmenter considérablement la quantité présente dans la solution aqueuse.

Cette solution aqueuse a alors une viscosité très faible analogue à celle de l'eau, même à très forte concentration en polymère. Un autre intérêt de ce système est qu'il peut libérer soudainement la substance dissoute lorsqu'il rencontre un milieu plus acide, c'est-à-dire une source de protons. Dans l'organisme, ce milieu de pH plus acide peut être trouvé, par exemple, au niveau des lysosomes si le système pénètre dans les cellules. La possibilité de libérer la substance occluse par simple déplacement de l'équilibre acide-base au niveau des groupes amine tertiaire même lorsque la différence de pH est très faible, est illustrée ci-après dans la partie expérimentale.

On a également découvert de façon surprenante que, comme cela est montré dans la partie expérimentale, le mécanisme de libération n'est pas fondamentalement altéré par la présence de sels dissous, ce qui est important pour les applications biomédicales, compte-tenu de la force ionique des fluides biologiques. Pour l'utilisation comme médicament, il convient d'utiliser comme polyamines tertiaires de départ des polyamines qui peuvent être facilement éliminées ou dégradées in vivo. Parmi les polymères facilement dégradables on citera notamment ceux qui comportent dans la chaîne des groupements ester, amide, ou thioéther (à raison d'au moins un tous les 10 ou 20 motifs, afin de donner des fragments capables de passer la barrière rénale pour élimination).

En outre il convient bien entendu de n'utiliser pour les applications médicamenteuses que des polymères dont la toxicité est suffisamment réduite. C'est le cas notamment des polymères partiellement quaternisés obtenus au départ des polymères particuliers mentionnés ci-dessus.

On mentionne ci-après à titre d'exemple la $DL_{50}$ de quelques polybases utilisables (la $DL_{50}$ varie avec le degré de quaternisation) :

- Q-P(TDAE) 20 : 85 mg/kg
- Q-P(BMAE) 19 : 178 mg/kg

L'invention a également pour objet un procédé de solubilisation de substances hydrophobes, caractérisé par le fait que l'on met en contact une polyamine tertiaire partiellement quaternisée et une substance hydrophobe (telles que définies ci-dessus) dans une phase aqueuse, de pH suffisamment élevé pour que ladite polyamine partiellement quaternisée se trouve sous forme globulaire.

Dans ces conditions, la substance hydrophobe se trouve incorporée dans les microglobules.

Ce procédé peut être utilisé par exemple pour le piégeage de faibles quantités de substances hydrophobes présentes dans la phase aqueuse. On peut ainsi séparer ladite substance hydrophobe du reste de la solution par exemple par ultrafiltration.

Le procédé de l'invention peut également être utilisé pour solubiliser ou disperser en phase aqueuse une substance hydrophobe en incorporant ladite substance hydrophobe dans des globules constitués par des polyamines tertiaires partiellement quaternisées. Pour cela on peut par exemple opérer de la façon suivante: on dissout la polyamine tertiaire partiellement quaternisée dans une solution aqueuse, à un pH correspondant à la conformation globulaire, on ajoute ensuite la substance hydrophobe en quantité supérieure à la quantité normalement soluble en l'absence du polymère et on élimine si nécessaire les produits non solubilisés.

Lors des modes d'exécution particuliers, ce procédé de l'invention peut encore présenter les caractéristiques suivantes prises isolément ou en combinaison:

- on effectue l'agitation avec un agitateur classique ou de préférence en soumettant la solution à l'action des ultrasons;
- on sépare les produits insolubles par filtration;
- on soumet la solution obtenue, si désiré, à une lyophilisation; on a en effet découvert qu'il est généralement possible de conserver sous forme lyophilisée la combinaison de la polyamine tertiaire partiellement quaternisée et de la substance hydrophobe; en général le produit lyophilisé se solubilise rapidement en solution aqueuse.

La figure 1 annexée représente un exemple de courbes de titrage potentiométrique obtenues avec certaines polyamines partiellement quaternisées, dans le cas des Q-PTDAE, en comparaison avec PTDAE. Dans la figure 1, $\delta$ représente le degré de neutralisation.

L'examen de ces courbes montre clairement l'effet tampon obtenu grâce aux polyamines tertiaires partiellement quaternisées.

Le plateau des courbes correspond à la zone où une partie des molécules de polyamine passe de la conformation globulaire à une conformation

déployée, ce qui permet la libération progressive, dans une zone de pH très étroite de la substance hydrophobe emprisonnée dans les microglobules. La zone de pH correspondant à la conformation globulaire est la zone de pH supérieur au pH correspondant au plateau.

Les exemples suivants illustrent l'invention sans toutefois la limiter.

EXEMPLE 1

Dissolution de la progestérone

A) Les essais sont effectués à partir d'une solution basique de poly(diéthylaminométhyl thiiranne) quaternisée à 19% Q-P(TDAE)19 de formule

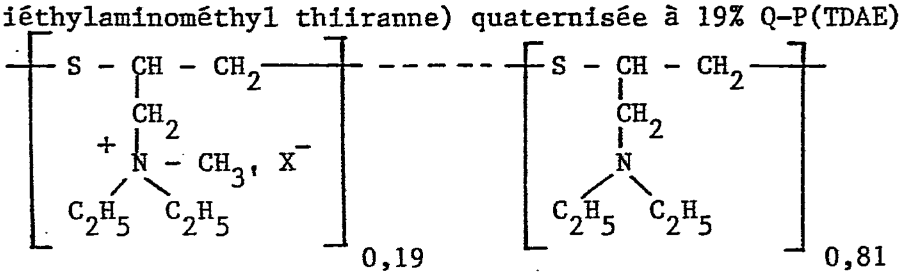

$X^-$ étant un anion (par exemple un anion halogénure tel que chlorure ou bromure), à une concentration $T_N = (2,5 \pm 0,1) \times 10^{-2}$ N (polymère = 3,77mg de polymère sous forme basique par ml de solution).

Dans le cas présent, $X^-$ représente un anion chlorure.

Cette solution est obtenue de la façon suivante:

270mg de chlorhydrate de Q-P(TDAE)19 sont dissous dans 10ml d'eau distillée, puis passés sur une résine échangeuse d'anions (BIO-RAD AG 1-x4-20-50 mesh-) sous forme $OH^-$ et récupérés dans une fiole jaugée de 50ml.

Des échantillons contenant 5ml de cette solution basique sont ajustés, par addition de HCl 0,5N, aux pH correspondant à différentes valeurs du degré de protonation apparent $\bar{\beta}$ de la fonction amine tertiaire résiduelle dans le domaine $0 < \bar{\beta} < 1$. On ajoute 5mg de progestérone en poudre à chaque échantillon et les mélanges sont soit soniqués pendant 5 minutes soit agités pendant 48 heures.

Après dissolution, chaque échantillon est filtré sur filtre millipore (0,45 micromètres). Les filtrats sont clairs et limpides. Le système "véhicule-drogue" peut être conservé de deux façons différentes:

- en solution, pendant quelques mois au réfrigérateur
- sous forme solide, en lyophilisant les filtrats et en les conservant sous vide. Le filtrat lyophilisé se solubilise rapidement en solution aqueuse.

Le dosage de la progestérone solubilisée ne peut être fait que par extraction de cette dernière des microémulsions. Cette extraction est obtenue par éclatement des véhicules globulaires dans un mélange filtrat-éthanol absolu dans le rapport V/V : 20/80. 1ml de chaque filtrat est dilué à 5ml par

addition d'éthanol. Les différents filtrats présentent dans la région 350-250nm des bandes dichroïques caractéristiques de la progestérone. Le signal de dichroïsme circulaire à $\lambda$ = 285nm est utilisable pour évaluer la quantité de progestérone présente dans chaque filtrat par comparaison avec les ellipticités de solutions standard de progestérone dissoute dans le même milieu solvant.

Le tableau suivant donne les quantités de progestérone solubilisée (en microgramme par ml de filtrat) en fonction du pH et du degré de protonation $\bar{\beta}$ pour chaque essai.

La solubilité de la progestérone dans l'eau pure est d'environ 11 à 12 microgrammes par ml.

| Essai | HCl 0,5N en ml | pH | $\bar{\beta}$ | progestérone (microgrammes/ml) |
|---|---|---|---|---|
| 1 | 0,063 | 7,5 | 0,07 | 75 |
| 2 | 0,075 | 6,97 | 0,135 | 62-64 |
| 3 | 0,088 | 6,95 | 0,20 | 48-50 |
| 4 | 0,10 | 6,95 | 0,26 | 38 |
| 5 | 0,125 | 6,95 | 0,38 | 26 |
| 6 | 0,175 | 6,80 | 0,63 | 14-16 |
| 7 | 0,20 | 6,55 | 0,75 | 11-12 |
| 8 | 0,23 | 6,15 | 0,88 | 11-12 |

EXEMPLE 2

On opère comme à l'exemple 1 mais les essais sont effectués à partir d'une concentration $T_N$ = 0,15N de Q-P(TDAE)19 sous forme basique. Des échantillons contenant 2ml de cette solution sont ajustés à différents $\bar{\beta}$ par addition de HCl N et additionnés de 5mg de progestérone. Après sonication pendant 5 minutes, les mélanges sont filtrés sur filtre millipore (0,45 micromètre). Le dosage de la progestérone solubilisée est fait comme précédemment. 0,2ml de chaque filtrat sont dilués à 2ml par de l'éthanol absolu. Les ellipticités sont mesurées à $\lambda$ = 285nm et comparées aux solutions standard de progestérone effectuées dans les mêmes conditions.

Le tableau suivant donne les quantités de progestérone solubilisée (en microgrammes par ml de filtrat) en fonction du pH et du degré de protonation $\bar{\beta}$.

| Essai | HCl 1 N en ml | pH | $\bar{\beta}$ | progestérone (microgrammes/ml) |
|---|---|---|---|---|
| 1 | 0,09 | 7,50 | 0,13 | 315 |
| 2 | 0,1 | 7,50 | 0,17 | 300 |
| 3 | 0,12 | 7,50 | 0,26 | 225 |
| 4 | 0,17 | 7,40 | 0,47 | 185-190 |
| 5 | 0,18 | 7,36 | 0,51 | 80 |
| 6 | 0,2 | 7,25 | 0,58 | 70 |
| 7 | 0,295 | 4,8 | 0,98 | 10 à 12 |

EXEMPLE 3

Deux essais sont réalisés dans les mêmes conditions: concentration en polymère, $\bar{\beta}$ et pH identiques mais on ajoute au deuxième essai, du chlorure de sodium de telle façon que la solution soit à 0,9% en NaCl.

Deux échantillons contenant 2ml de solution basique de Q-P(TDAE)19 ($T_N$ = 0,125 N) sont ajustés à pH = 7,7 et à $\bar{\beta}$ = 0,06 par addition de 0,06ml de HCl N. Dans le deuxième échantillon, on ajoute 18mg de NaCl. 5mg de progestérone sont additionnés à chaque échantillon. Les mélanges sont soniqués pendant 5 minutes. Le protocole de filtration et de dosage de la progestérone solubilisée est le même qu'à l'exemple 2.

La quantité de progestérone dissoute dans chaque essai est de 375 microgrammes par ml de filtrat.

EXEMPLE 4

Dissolution de l'estradiol

Les essais sont faits comme à l'exemple 1 à partir d'une solution basique de Q-P(TDAE)19 ($T_N = 2,5 \times 10^{-2}$ N).

Des échantillons contenant 5ml de cette solution basique sont ajustés à différents $\bar{\beta}$ par addition de HCl 0,5N. On ajoute 4mg d'estradiol en poudre dans chaque essai et on laisse soniquer pendant 5 minutes ou agiter pendant 48 heures. La filtration sur filtre millipore (0,45 micromètre) donne des solutions limpides qui peuvent être conservées de la même façon que dans l'exemple 1. Le dosage de l'estradiol solubilisé dans les microémulsions ne peut se faire qu'en milieu acide qui provoque l'éclatement des globules. La drogue libérée précipite alors dans la solution sous forme d'un léger louche blanc.

A 1ml de filtrat on ajoute 1 goutte de HCl N et on dilue à 5ml par de l'éthanol absolu. Les ellipticités sont mesurées à $\lambda$ = 228-229nm et comparées aux ellipticités de solutions standard d'estradiol dissoutes dans le

même milieu solvant ($H_2O$/Ethanol - 20/80 - V/V).

Le tableau suivant donne les quantités d'estradiol (en microgrammes par ml de filtrat) dissous en fonction du pH et du degré de protonation $\bar{\beta}$ des fonctions amine tertiaire résiduelles. La solubilité de l'estradiol dans l'eau est d'environ 1 microgramme par ml.

| Essai | HCl 0,5N en ml | pH | $\bar{\beta}$ | estradiol (microgrammes/ml) |
|---|---|---|---|---|
| 1 | 0,05 | 7,5 à 7,6 | 0,02 | 45 à 50 |
| 2 | 0,068 | 7,2 | 0,1 | 38 |
| 3 | 0,093 | 6,97 | 0,22 | 26 |
| 4 | 0,103 | 6,95 | 0,27 | 18 |
| 5 | 0,118 | 6,95 | 0,35 | 11 |
| 6 | 0,138 | 6,92 | 0,45 | 5 |
| 7 | 0,165 | 6,74 | 0,58 | |
| 8 | 0,195 | 6,59 | 0,73 | 1 à 2 |
| 9 | 0,233 | 6,0 | 0,93 | |
| 10 | 0,253 | 4,30 | 1,01 | |

EXEMPLE 5

Dissolution de la griseofulvine

Les essais sont effectués à partir d'une solution basique de Q-P(TDAE)19 ($T_N$= 2,5 x $10^{-2}$ N). Des échantillons contenant 5ml de cette solution basique sont ajustés aux pH correspondant aux différents $\bar{\beta}$ par addition de HCl 0,5N).

7mg de griseofulvine en poudre sont ajoutés à chaque échantillon. On laisse soniquer pendant 5 minutes et on filtre les mélanges sur filtre millipore (0,45 micromètre). Les filtrats limpides sont conservés de la même façon que pour les exemples 1 et 2. Le dosage de la griseofulvine solubilisée est effectué dans le milieu solvant eau-acétonitrile : 20/80, V/V. On prélève dans chaque essai, 1ml de filtrat qui est dilué à 5ml par de l'acétonitrile. Les ellipticités des différentes solutions sont mesurées à $\lambda$ = 294nm et comparées à des solutions standard de griseofulvine dissoutes dans le même milieu solvant.

Le tableau suivant donne les quantités de griseofulvine dissoute (en microgrammes par ml de filtrat) en fonction du pH et du degré de protonation $\bar{\beta}$ . La solubilité de la griseofulvine dans l'eau est de l'ordre de 13 à 15 microgrammes par ml.

| Essai | HCl 0,5N en ml | pH | $\overline{\beta}$ | griseofulvine (microgrammes/ml) |
|---|---|---|---|---|
| 1 | 0,052 | 7,6 | 0,02 | 47 |
| 2 | 0,067 | 7,08 | 0,093 | 43 |
| 3 | 0,085 | 7,0 | 0,185 | 36 |
| 4 | 0,10 | 6,95 | 0,26 | 30 |
| 5 | 0,113 | 6,95 | 0,32 | 26 |
| 6 | 0,133 | 6,95 | 0,42 | 22 |
| 7 | 0,15 | 6,83 | 0,51 | 18 |
| 8 | 0,184 | 6,67 | 0,673 | 15 |
| 9 | 0,228 | 6,20 | 0,89 | 15 |
| 10 | 0,25 | 4,5 à 4,6 | 1 | 15 |

REVENDICATIONS DE BREVET

1. Composition sous forme de microglobules solubles dans l'eau, caractérisée par le fait que lesdits globules sont constitués par au moins une polyamine tertiaire partiellement quaternisée, que dans lesdits microglobules est incorporée au moins une substance hydrophobe, et que ladite polyamine tertiaire partiellement quaternisée dérive d'une polyamine tertiaire insoluble dans l'eau, soluble en milieu acide, et présentant un fort pouvoir tampon en milieu aqueux.

2. Composition selon la revendication 1, caractérisée par le fait que la proportion pondérale de substance hydrophobe est de 0,01 à 100 %, par rapport à ladite polyamine partiellement quaternisée.

3. Composition selon la revendication 1 ou 2, caractérisée par le fait que le degré de quaternisation de la polyamine tertiaire est d'au moins 2 %.

4. Composition selon la revendication 3, caractérisée par le fait que le degré de quaternisation de la polyamine tertiaire va de 3 % à 30 %.

5. Composition selon l'une quelconque des revendications précédentes, caractérisée par le fait que ladite polyamine tertiaire partiellement quaternisée a un poids moléculaire supérieur à 5000.

6. Composition selon l'une quelconque des revendications précédentes, caractérisée par le fait que ladite polyamine tertiaire est choisie parmi les poly[(N,N-dialkylaminoalkyl) éthylène], les poly [thio (N,N-dialkylaminoalkyl)-1 éthylène] et les poly [(N,N-dialkylaminoalkyl acrylates (ou méthacrylates)].

7. Composition selon l'une quelconque des revendications 1 à 5, caractérisée par le fait que ladite polyamine tertiaire est composée de motifs de formule :

$$-\ NH-(CH_2)_4-\underset{\substack{|\\ N(Alk.)_2}}{CH}-CO-$$

dans laquelle Alk. est un groupement alkyle ayant 1 à 4 atomes de carbone.

8. Composition selon l'une quelconque des revendications précédentes, caractérisée par le fait qu'elle se présente soit sous la forme d'une solution aqueuse soit sous la forme d'un produit lyophilisé.

9. Procédé de solubilisation de substances hydrophobes, caractérisé par le fait que l'on met en contact une polyamine tertiaire partiellement quaternisée dérivant d'une polyamine tertiaire telle que définie dans la revendication 1 et une substance hydrophobe dans une phase aqueuse de pH

suffisamment élevé pour que ladite polyamine partiellement quaternisée se trouve sous forme globulaire.

10. Procédé selon la revendication 9, caractérisé par le fait que l'on dissout la polyamine tertiaire partiellement quaternisée dans une solution aqueuse contenant en suspension la substance hydrophobe, que l'on agite et que l'on sépare les microglobules, dans lesquelles s'est incorporée la substance hydrophobe, du reste de la solution selon les méthodes connues.

11. Procédé selon la revendication 9, caractérisé par le fait que l'on dissout la polyamine tertiaire partiellement quaternisée dans une solution aqueuse, à un pH correspondant à la conformation globulaire, que l'on ajoute ensuite la substance hydrophobe en quantité supérieure à la quantité normalement soluble en l'absence du polymère, et on élimine si nécessaire l'excès de substance hydrophobe non solubilisée.

12. Procédé selon la revendication 11, caractérisé par le fait que l'on soumet ensuite la solution obtenue à une lyophilisation.

13. Procédé selon l'une quelconque des revendications 9 à 12, caractérisé par le fait que le degré de quaternisation de la polyamine tertiaire est d'au moins 2 %.

14. Procédé selon l'une quelconque des revendications 9 à 13, caractérisé par le fait que le degré de quaternisation de la polyamine tertiaire va de 3 % à 30 %.

15. Procédé selon l'une quelconque des revendications 9 à 14, caractérisé par le fait que ladite polyamine tertiaire insoluble dans l'eau comporte des groupements amine tertiaire aliphatiques ou aromatiques.

16. Procédé selon l'une quelconque des revendications 9 à 15, caractérisé par le fait que ladite polyamine tertiaire est choisie parmi les poly /(N,N-dialkylaminoalkyl) éthylène7, les poly/thio(N,N-dialkylamino-alkyl)-1 éthylène7et les poly/(N,N-dialkylaminoalkyl acrylates (ou méthacrylates)7.

17. Procédé selon l'une quelconque des revendications 9 à 15, caractérisé par le fait que ladite polyamine tertiaire est telle que définie dans la revendication 7.

Office européen des brevets

**RAPPORT DE RECHERCHE EUROPEENNE**

0120757

Numéro de la demande

EP 84 40 0481

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X,Y | JOURNAL OF THE AMERICAN CHEMICAL SOCIETY, vol. 102, no. 21, 8 octobre 1980, pages 6638-6440, American Chemical Society S.L. REGEN et al.: "Polymerized vesicles"<br>* Article entier *<br>--- | 1-17 | A 61 K 9/50 |
| D,Y | POLYMER JOURNAL, vol. 12, no. 2, 1980, pages 113-124 D. VALLIN et al.: "Partial methylation of poly[thio-1-(N,N-dimethylaminomethyl)ethylene] and conformational behavior of resulting dibasic polyelectrolytes"<br>* Page 113, colonnes 1,2; page 114, haut des colonnes 1,2; pages 123-124, conclusion *<br>--- | 2-6,12-16 | DOMAINES TECHNIQUES RECHERCHES (Int. Cl. 3) |
| D,Y | POLYMER JOURNAL, vol. 14, no. 5, 1982, pages 335-342 J. HUGUET et al.: "Acid-base and chiroptical properties of partially quaternized optically active poly[thio-1-(N-(S)-s-butyl-N-methylaminomethyl)ethylene]<br>* Page 341, colonne 1, 3 dernières lignes - colonne 4, fin *<br>--- -/- | 2-6,12-16 | A 61 K 9/00 |

Le présent rapport de recherche a été établi pour toutes les revendications

| Lieu de la recherche | Date d'achèvement de la recherche | Examinateur |
|---|---|---|
| LA HAYE | 05-06-1984 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503. 03.82

0120757

Office européen des brevets

RAPPORT DE RECHERCHE EUROPEENNE

Numéro de la demande

EP 84 40 0481



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,Y | DIE MAKROMOLEKULARE CHEMIE, vol. 170, 1973, pages 23-37 J. HUGUET et al.: "Polymerization of oxiranylalkylamines and thiiranylalkylamines derived from achiral and racemic secondary amines" * Page 24, 5 dernières lignes - page 25, haut de la page table 1 * | 2-6,12 -16 | |
| | ----- | | DOMAINES TECHNIQUES RECHERCHES (Int. Cl. 3) |

Le présent rapport de recherche a été établi pour toutes les revendications

| Lieu de la recherche | Date d'achèvement de la recherche | Examinateur |
|---|---|---|
| LA HAYE | 05-06-1984 | BERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82